# EUROPÄISCHE PATENTANMELDUNG

(11) **EP 1 987 816 A1**
(43) Veröffentlichungstag der Anmeldung: **05.11.2008**
(21) Anmeldenummer: 07008755.6
(22) Anmeldetag: 30.04.2007
(51) Int. Cl.: A61K 9/14, A61K 31/00

(54) **Adsorbate eines Rasagilinsalzes mit einem wasserlöslichen Hilfsstoff**

(71) Anmelder: Ratiopharm GmbH, 89079 Ulm (DE)
(72) Erfinder: Lehmann, Alexander, 03238 Zürchel (DE); Muskulus, Frank, 82194 Gröbenzell (DE); Schulze Nahrup, Julia, 82061 Neuried (DE)
(74) Vertreter: Best, Michael

(57) **Zusammenfassung**

Die Erfindung betrifft ein Adsorbat eines pharmazeutisch verträglichen Salzes des Rasagilins mit zumindest einem pharmazeutisch verträglichen Hilfsstoff, wobei der zumindest eine pharmazeutisch verträgliche Hilfsstoff ein wasserlöslicher, organischer Hilfsstoff ist und das Salz des Rasagilins in dem Adsorbat als amorphe Substanz vorliegt.

## Beschreibung

Die vorliegende Erfindung betrifft neue Adsorbate von pharmazeutisch verträglichen Salzen des bekannten Wirkstoffs Rasagilin mit wasserlöslichen, pharmazeutisch verträglichen, organischen Hilfsstoffen. In diesen neuen Adsorbaten liegt das pharmazeutisch verträgliche Salz des Rasagilins in einer amorphen Form vor, die stabil ist und sich auch bei langer Lagerung nach Einarbeitung des Adsorbats in ein Arzneimittel nicht in eine andere, z.B. kristalline Form des pharmazeutisch verträglichen Salzes des Rasagilins umwandelt.

Rasagilin ist die Bezeichnung für die chemische Verbindung R(+)-N-Propargyl-1-aminoindan, das auch als R(+)PAI bezeichnet wird.

Rasagilin ist ein seit langem bekannter MAO-Inhibitor, der als Wirkstoff zur Behandlung einer Reihe von Erkrankungen eingesetzt wird. So offenbart das US 5,532,415 die Herstellung des Rasagilins und seiner Salze sowie die Anwendung des Wirkstoffs zur Behandlung einer Reihe von Erkrankungen, z.B. von Parkinson, Erinnerungsstörungen, Demenz, Depression, Schizophrenie, Hyperaktivität, etc. Ein Verfahren zur Herstellung von Salzen des Rasagilins ist ebenfalls in der WO 02/068376 offenbart.

Auch wenn Rasagilin und seine Salze bereits seit längerem als pharmazeutische Wirkstoffe beschrieben sind, bereitet die Formulierung von Arzneimitteln mit pharmazeutisch verträglichen Salzen des Rasagilins Schwierigkeiten. So beschreibt die US-A 6,126,968, dass pharmazeutische Formulierungen mit PAI, das entweder als Racemat oder als (+)- oder als (-)-Enantiomer vorliegen kann, Probleme mit der Stabilität aufweisen, und zur Lösung dieser Probleme schlägt die US-A 6,126,968 vor, die Wirkstoffe mit einer sehr großen Menge eines organischen Alkohols wie insbesondere Mannit zu formulieren. In der WO 2006/091657 wird von Problemen berichtet, die dadurch auftreten, dass die arzneimittelrechtlich erforderliche gleichmäßige Verteilung eines Rasagilinsalzes in einem Arzneimittel nicht gut gewährleistet werden kann. Zur Lösung dieses Problems schlägt die WO 2006/091657 vor, Wirkstoffteilchen einer bestimmten Größe zu verwenden.

Von zunehmender Bedeutung im Bereich der Arzneimittelformulierungen ist es ebenfalls, dass sich die physikalische Form eines Wirkstoffs (also die polymorphe oder amorphe Form) während der Lagerzeit der Arzneimittelformulierung nicht verändert und die Arzneimittel den Wirkstoff in einer definierten und reproduzierbaren polymorphen oder amorphen Form enthalten. Die pharmazeutisch verträglichen Salze des Rasagilins liegen üblicherweise in kristalliner Form vor, das Auftreten von Polymorphen ist daher sehr wahrscheinlich. Werden solche polymorphen Formen in Arzneimittel eingearbeitet, ist es vorteilhaft, dass eine reine polymorphe Form verwendet wird, und es muss sichergestellt werden, dass keine Umwandlung zwischen den einzelnen Polymorphen in dem Arzneimittel auftritt. Daher wäre es vorteilhaft, Rasagilin in einer amorphen Form zur Verfügung zu stellen, die reproduzierbar erhältlich und gut verarbeitbar ist und sich nicht in andere Formen umwandelt. Es war bislang allerdings noch nicht möglich, die pharmazeutisch verträglichen Salze des Rasagilins reproduzierbar in einer stabilen amorphen Form zur Verfügung zu stellen, die für die Formulierung von Arzneimitteln geeignet ist.

Aufgabe der Erfindung ist es daher, pharmazeutisch verträgliche Salze des Rasagilins in einer Form zur Verfügung zu stellen, so dass nach Einarbeitung in ein Arzneimittel der Wirkstoff über die Lagerzeit des Arzneimittels stabil ist, es zu keinen Umwandlungen in andere physikalische Formen des Wirkstoffs kommt und auf einfache Art und Weise eine Homogenität der Wirkstoffverteilung in den Arzneimitteln sichergestellt werden kann. Die Form der pharmazeutisch verträglichen Salze des Rasagilins sollte außerdem leicht hergestellt und gut verarbeitet werden können.

Die Lösung dieser Aufgabe beruht auf dem überraschenden Befund, dass wässrige Lösungen, die ein pharmazeutisch verträgliches Salz des Rasagilins und einen wasserlöslichen, organischen, pharmazeutisch verträglichen Hilfsstoff enthalten, nach dem Entfernen des Lösungsmittels Adsorbate zwischen dem pharmazeutisch verträglichen Salz des Rasagilins und dem pharmazeutisch verträglichen, organischen Hilfsstoff bilden, bei denen das pharmazeutisch verträgliche Salz des Rasagilins in amorpher Form vorliegt. Diese Adsorbate lassen sich sehr leicht verarbeiten, sind sehr stabil in pharmazeutischen Formulierungen, insbesondere in Tabletten, Kapseln, Pellets und Granulat, sie liefern Arzneimittel mit einer hohen Gleichförmigkeit der Wirkstoffverteilung, und insbesondere ändert sich die amorphe Form der Rasagilinsalze nach der Einarbeitung in Arzneimittel und während der Lagerung der Arzneimittel nicht zu anderen physikalischen Formen.

Die Erfindung stellt daher ein Adsorbat eines pharmazeutisch verträglichen Salzes des Rasagilins mit zumindest einem pharmazeutisch verträglichen Hilfsstoff zur Verfügung, wobei der zumindest eine pharmazeutisch verträgliche Hilfsstoff ein wasserlöslicher, organischer Hilfsstoff ist und das Salz des Rasagilins in dem Adsorbat als amorphe Substanz vorliegt. Die Erfindung stellt ebenfalls ein Verfahren zur Herstellung derartiger Adsorbate und Arzneimittel, die diese Adsorbate enthalten, zur Verfügung.

Die erfindungsgemäßen Adsorbate enthalten bevorzugt keinen anorganischen Hilfsstoff.

Pharmazeutisch verträgliche Salze des Rasagilins fallen bei Ihrer Herstellung in der Regel als kristalline Substanz an. Löst man diese Salze in Wasser (oder einem wässrigen Lösemittel) und entfernt das Wasser, beispielsweise durch Sprühtrocknung, entstehen wiederum kristalline oder teilkristalline Substanzen. Überraschend wurde nun gefunden, dass in dem Fall, in dem ein pharmazeutisch verträgliches Salz des Rasagilins mit einem organischen, wasserlöslichen, pharmazeutisch verträglichen Hilfsstoff aufgelöst wird und anschließend das Wasser entfernt wird, z.B. durch Sprühtrocknung, ein Adsorbat zwischen dem pharmazeutisch verträglichen Salz des Rasagilins und dem organischen, wasserlöslichen, pharmazeutisch verträglichen Hilfsstoff entsteht, in dem das pharmazeutisch verträgliche Salz des Rasagilins in amorpher Form vorliegt.

Unter amorpher Form wird erfindungsgemäß eine Form des Wirkstoffs verstanden, bei der in einem Röntgendiffraktogramm die Beugungsreflexe des Wirkstoffs nicht mehr auftreten. Zu Einzelheiten der Bestimmung des Röntgendiffraktogramms wird auf die nachfolgenden Beispiele verwiesen.

Die pharmazeutisch verträglichen Salze des Rasagilins sind nicht besonders eingeschränkt, hierfür können alle üblicherweise verwendeten, pharmazeutisch verträglichen Salze eingesetzt werden. Bevorzugte Salze sind die Tatrate, die Esylate, die Mesylate, die Edisilate und die Sulfate, am stärksten bevorzugt ist das Mesylatsalz des Rasagilins.

In einer bevorzugten Ausführungsform der Erfindung besteht das Adsorbat aus dem pharmazeutisch verträglichen Salz des Rasagilins und einem oder mehreren, bevorzugt einem, pharmazeutisch verträglichen, wasserlöslichen, organischen Hilfsstoff. In dieser Ausführungsform enthält das Adsorbat keine wasserunlöslichen Bestandteile und keine anorganischen Bestandteile.

In einer weiteren bevorzugten Ausführungsform besteht das Adsorbat aus dem pharmazeutisch verträglichen Salz des Rasagilins und einem oder mehreren, bevorzugt einem, pharmazeutisch verträglichen, organischen, wasserlöslichen Hilfsstoff und einem oder mehreren, bevorzugt einem wasserunlöslichen Hilfsstoff. Der wasserunlösliche Hilfsstoff ist bevorzugt ebenfalls eine organische Verbindung, die Anwesenheit von anorganischen Verbindungen in den Adsorbaten ist erfindungsgemäß nicht bevorzugt.

Falls wasserunlösliche, pharmazeutisch verträgliche Hilfsstoffe Bestandteil des Adsorbats sind, handelt es sich hierbei bevorzugt um wasserunlösliche, pharmazeutisch verträgliche Hilfsstoffe mit einer Teilchengröße von 150 µm oder darunter, besonders bevorzugt von 100 µm oder darunter, insbesondere im Bereich von ca. 50 µm. Die Untergrenze der Teilchengröße der pharmazeutisch verträglichen, wasserunlöslichen Hilfsstoffe liegt bei etwa 10 µm, stärker bevorzugt bei etwa 20 µm. Besonders bevorzugt handelt es sich um mikrokristalline Cellulose, insbesondere bevorzugt um mikrokristalline Cellulose mit einer mittleren Teilchengröße von 150 µm oder weniger, stärker bevorzugt von 100 µm oder weniger, insbesondere von etwa 50 µm. Ganz besonders bevorzugt ist die mikrokristalline Cellulose Avicel PH101.

Wesentlicher Bestandteil der erfindungsgemäßen Adsorbate ist zumindest ein pharmazeutisch verträglicher Hilfsstoff, bei dem es sich um einen wasserlöslichen, organischen Hilfsstoff handelt. Bevorzugt enthält das erfindungsgemäße Adsorbat einen wasserlöslichen, organischen, pharmazeutisch verträglichen Hilfsstoff, es ist aber auch möglich, dass mehrere wasserlösliche, pharmazeutisch verträgliche, organische Hilfsstoffe eingesetzt werden, z.B. zwei, drei oder vier derartige Hilfsstoffe.

Bei den pharmazeutisch verträglichen, wasserlöslichen, organischen Hilfsstoffen handelt es sich bevorzugt um wasserlösliche Polymere, z.B. solche, wie sie im Stand der Technik als Bindemittel für Arzneimittel eingesetzt werden, z.B. um Celluloseether oder Polyvinylpyrrolidon. Besonders bevorzugt handelt es sich um Hydroxypropylmethylcellulose, Hydroxypropylcellulose oder Polyvinylpyrrolidon. Falls als wasserlöslicher, organischer, pharmazeutisch verträglicher Hilfsstoff ein derartiges wasserlösliches Polymer eingesetzt wird, weist das Adsorbat bevorzugt keinen wasserunlöslichen Hilfsstoff auf.

Ein weiterer bevorzugter wasserlöslicher Hilfsstoff ist eine organische Säure, wie insbesondere eine organische Mono-, Di- oder Tricarbonsäure mit bis zu zehn Kohlenstoffatomen, die gegebenenfalls und bevorzugt ein bis drei Hydroxylgruppen enthält. Die am stärksten bevorzugte organische, wasserlösliche Säure ist Zitronensäure. Falls der organische, pharmazeutische, wasserlösliche Hilfsstoff kein wasserlösliches Polymer darstellt, sondern beispielsweise eine organische, wasserlösliche Säure, wie Zitronensäure, ist es bevorzugt, dass dieser organische, wasserlösliche, pharmazeutisch verträgliche Hilfsstoff zusammen mit einem wasserunlöslichen, pharmazeutisch verträglichen Hilfsstoff wie vorstehend definiert verwendet wird. Anstelle der organischen Säure kann selbstverständlich auch ein Salz dieser Säure verwendet werden.

Das Verhältnis zwischen dem pharmazeutisch verträglichen Salz des Rasagilins und dem zumindest einen pharmazeutisch verträglichen, wasserlöslichen, organischen Hilfsstoff in dem Adsorbat ist bevorzugt im Bereich von 5:1 bis 1:20, stärker bevorzugt im Bereich von 2:1 bis 1:15. Am stärksten bevorzugt ist es, dass die Menge an pharmazeutisch verträglichem, wasserlöslichem, organischem Hilfsstoff mindestens so groß oder größer ist, als die Menge an pharmazeutisch verträglichem Salz des Rasagilins, so dass das Verhältnis zwischen dem Salz und dem Hilfsstoff am stärksten bevorzugt im Bereich von 1:1 bis 1:10 liegt.

Falls in dem Adsorbat mehr als ein pharmazeutisch verträglicher, wasserlöslicher, organischer Hilfsstoff vorhanden ist, gelten die vorstehenden Angaben zu dem Verhältnis zwischen dem pharmazeutisch verträglichen Salz des Rasagilins und dem zumindest einen pharmazeutisch verträglichen, wasserlöslichen, organischen Hilfsstoff ebenfalls für die bevorzugten Verhältnisse zwischen dem pharmazeutisch verträglichen Salz des Rasagilins und der Summe aller pharmazeutisch verträglicher, wasserlöslicher, organischer Hilfsstoffe in dem Adsorbat. Falls in dem erfindungsgemäßen Adsorbat ein oder mehrere wasserunlösliche, pharmazeutisch verträgliche Hilfsstoffe vorhanden sind, ist das Verhältnis zwischen dem pharmazeutisch verträglichen Salz des Rasagilins und dem einen oder mehreren (bevorzugt einen) pharmazeutisch verträglichen, wasserunlöslichen Hilfsstoffen bevorzugt im Bereich von 5:1 bis 1:20, stärker bevorzugt im Bereich von 2:1 bis 1:15 und am stärksten bevorzugt im Bereich von 1:1 bis 1:10.

Im Rahmen dieser Anmeldung sind alle Verhältnisangaben, alle Prozentangaben und alle Gehaltsangaben auf das Gewicht bezogen, sofern nichts anderes ausdrücklich offenbart oder aufgrund der Umstände für einen Fachmann ohne weiteres offensichtlich ist.

Ein Hilfsstoff ist im Sinne dieser Anmeldung wasserlöslich, wenn mehr als 0,1 mg des Hilfsstoffs in 1 ml Wasser gelöst werden kann. Bevorzugt ist ein wasserlöslicher Hilfsstoff derart löslich, dass mehr als 1 mg, stärker bevorzugt mehr als 10 mg, insbesondere mehr als 100 mg, z.B. 1000 mg oder mehr in 1 ml Wasser gelöst werden kann.

Ein Hilfsstoff ist im Sinne dieser Anmeldung wasserunlöslich, wenn höchstens 0,1 mg des Wirkstoffs in 1 ml Wasser gelöst werden kann.

Die Löslichkeiten beziehen sich jeweils auf destilliertes Wasser bei 25 °C und pH = 7.

Die erfindungsgemäßen Adsorbate können auf einfache Art und Weise hergestellt werden, indem ein pharmazeutisch verträgliches Salz des Rasagilins und der zumindest eine organische, wasserlösliche, pharmazeutisch verträgliche Hilfsstoff sowie gegebenenfalls weitere wasserlösliche Bestandteile des Adsorbats in Wasser oder einem wässrigen Lösemittel gelöst werden. Falls wasserunlösliche Bestandteile in dem Adsorbat vorhanden sein sollten, werden diese wasserunlöslichen Bestandteile anschließend (oder vorher) in dem Lösemittel dispergiert. Anschließend wird das Lösemittel entfernt, bevorzugt durch eine Sprühtrocknung. Die Sprühtrocknung kann auf übliche Art und Weise durchgeführt werden, beispielsweise mit einem Sprühtrockner der Firma Büchi vom Typ B-191, beispielsweise bei einer Temperatur von 130°C und einer Sprührate von 5, 10 oder 20%. Die Sprühbedingungen sind jedoch nicht kritisch, solang keine Temperatur verwendet wird, bei der sich das Rasagilin während der Sprühzeit zersetzt.

Unter einem wässrigen Lösemittel wird erfindungsgemäß ein Lösemittel verstanden, das zu mindestens 50%, stärker bevorzugt zu mindestens 70% aus Wasser besteht, wobei der Rest aus einem oder mehreren mit Wasser mischbaren Lösemitteln besteht, insbesondere aus einem Alkohol wie Ethanol oder Isopropanol. Bevorzugt wird bei dem erfindungsgemäßen Verfahren zur Herstellung der erfindungsgemäßen Adsorbate Wasser als Lösemittel eingesetzt.

Die erfindungsgemäßen Adsorbate können auf übliche Art und Weise zu Arzneimitteln weiterverarbeitet werden. Falls gewünscht, können die Adsorbate auf eine vorteilhafte Teilchengröße vermahlen werden und auf eine vorteilhafte Teilchenverteilung gesiebt werden.

Die erfindungsgemäßen Adsorbate können generell zur oralen, parenteralen, rektalen oder transdermalen Verabreichung hergerichtet sein, bevorzugt zur oralen Verabreichung. Geeignete Darreichungsformen sind beispielsweise, Tabletten (durch Granulierung oder Direktverpressung hergestellt), im Mund zerfallende Tabletten, verzögert freisetzende Tabletten, Pellets oder Granulate, die beispielsweise in Dragees, Sachets, Hart- oder Weichgelantinekapseln, etc. eingebracht werden. Die Verarbeitung zu Tabletten, Pellets oder Granulat erfolgt dann wie es im Stand der Technik beschrieben wird und dem Fachmann bekannt ist, wobei beispielsweise auf das Standardlehrbuch "Die Tablette", Wolfgang A. Ritschel und Anette Bauer-Brandl, 2. Auflage, Editio Cantor Verlag, 2002, verwiesen werden kann.

Die erfindungsgemäßen Tabletten, Pellets oder Granulen können beispielsweise geeignete Träger (bzw. Füllmittel) Bindemittel, Schmiermittel, Sprengmittel, Farbstoffe, Geschmacksmittel, Fließstoffe, Filmüberzüge und gegebenenfalls Schmelzmittel und weitere übliche Hilfs- und Zusatzstoffe enthalten. Geeignete Träger sind z.B. Pellets auf Zuckerbasis und mikrokristalliner Basis, Laktose, Alpha-Laktose-Monohydrat, Gelantine, Agar, Stärke, Maisstärke, Saccharose, Glukose, Methylcellulose, Dicalciumphosphat, Calciumphosphat, Calciumsilikate, Mannit, Sorbit, mikrokristalline Cellulose, Cellulosederivate, Fettsäure, Fette, Stearate, Öle, Wachse, Parafine, etc. Geeignete Bindemittel schließen Stärke, Gelatine, natürliche Zucker wie Glukose oder β-Laktose, Stärke, Gummi arabicum, Tragant, Natriumalginat, Povidon, Carboxymethylcellulose, Polyethylenglykol, Wachs, Hydroxypropylmethylcellulose, Hydroxypropylcellulose etc. ein. Schmiermittel sind beispielsweise Natriumoleat, Natriumstearat, Magnesiumstearat, Natriumbenzoat, Natriumacetat, Natriumchlorid, Stearinsäure, Natriumstearylfumarat, Talk, etc. Sprengmittel schließen die bekannten "super disintegrants" ein, aber auch Stärke, Methylcellulose, Agar, Bentonit, Xanthangummi, Pyrrolidone etc. können genannt werden. Besonders bevorzugt sind Natriumcrosscarmelose und Natriumstärkeglykolat. Als Filmüberzüge sowie funktionelle magensaftresistente und retardierende Filmüberzüge eignen sich Cellulosen und deren Derivate, Polyglycole, Povidone, Acrylpolymere, Copolimerisate auf Basis des Ethylacrylats, Methylmethacrylats, Polyvinylacetats und der Methacrylsäure. Weiterhin können die Arzneimittel zum Beispiel geeignete Farbstoffe auf Eisenoxidbasis enthalten.

Es war bereits bekannt, pharmazeutisch verträgliche Salze des Rasagilins mit den vorstehenden Hilfsstoffen zu formulieren. Unter diesen Hilfsstoffen finden sich auch pharmazeutisch verträgliche, wasserlösliche, organische Verbindungen. Durch die Verarbeitung dieser pharmazeutisch verträglichen, wasserlöslichen, organischen Verbindungen mit einem pharmazeutisch verträglichen Salz des Rasagilins entstehen aber nicht die erfindungsgemäßen Adsorbate, die daher neue Verbindungen sind. Damit solche Adsorbate erhalten werden, ist es nicht ausreichend, die pharmazeutisch verträglichen Salze des Rasagilins auf übliche Art und Weise mit Hilfsstoffen zu Arzneimitteln zu verarbeiten, sondern es muss eine Lösung des pharmazeutisch verträglichen Salzes des Rasagilins und der pharmazeutisch verträglichen, wasserlöslichen, organischen Hilfsstoffe, die das Adsorbat bilden sollen, gebildet werden, und anschließend muss das Wasser zur Bildung des Adsorbats entfernt werden. Die so hergestellten Adsorbate sind neu und im Stand der Technik noch nicht beschrieben.

Die folgenden Beispiele erläutern die Erfindung.

In den Beispielen wurde die Amorphisierung durch Röntgendiffraktometrie überprüft. Die Messungen werden ausgeführt an einem Bruker Advance D8, mit Theta-2Theta Goniometer (435 mm Durchmesser) in Reflexions-Geometrie (Bragg-Brentano). Die Strahlungsleistung der Röntgenröhre (Kupferanode Kα1, λ=1,5406 Å / Kα2, λ=1,54439 Å) wird bei einer Generatorleistung 40 KV/40 mA, und Divergenzschlitz 0,6 mm, gewählt. Als Ortsempfindlicher-Detektor (position sensitive Detector PSD), kommt ein Väntec-1 Detektor (12° 2⊖ Detektor Öffnungswinkel) zur Anwendung. Röntgenbeugungen werden aufgenommen im Bereich 3° 2⊖ bis 55° 20, mit einer Schrittweite von 0,016° 2⊖ (steps 2465) steptime: 75 ns. Zur Filterung der Cu-Kβ-Strahlung λ=1,39222 Å) wird ein sekundäres Nickelfilter (0,1 mm) verwendet. Primärer und sekundärer Soller-Spalt (2,5°), Antiscattering Schlitz 5,59 (fixed, Abstand zu Detektorfenster=110,8 mm). Detektorschlitz (statisch) 10,28 mm, Abstand zu Detektorfenster=21,3 mm).

Die Proben werden mit einem horizontalen Probenwechsler (9 Proben) zugeführt. Zur Überprüfung des ordnungsgemäßen Betriebs der Messanordnung wird vor jeder Messserie eine Korund-Standard-Probe vermessen. Die Intensitäten (α1/α2=0,5) und Reflexlagen, sowie die α1-a2-Reflexabstände dieser Messung werden mit einem Referenz-Beugungsdiagramm überlagert. Diese Auswertung und die Auswertung der Proben-Messergebnisse, sowie die weitere Bearbeitung der Rohdaten erfolgt mit dem Programmpaket EVA (Bruker).

Für die folgenden Versuche wurde Rasagilinmesylat verwendet, das entsprechend der US 5,532,415 hergestellt worden war.

### Referenzbeispiel 1

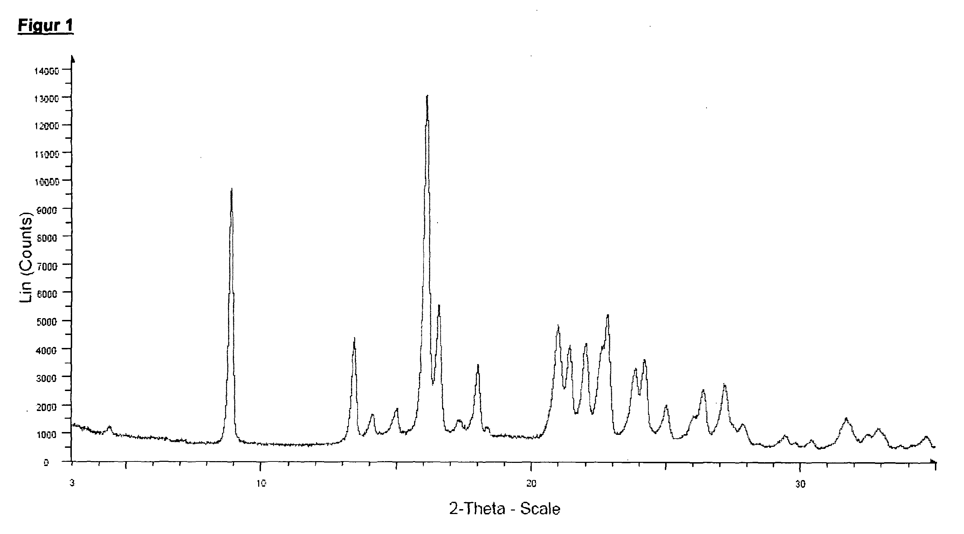

2 g Rasagilinmesylat wurden in 50 g Wasser in einem Becherglas gelöst und mit dem Sprühtrockner der Firma Büchi vom Typ B-191 sprühgetrocknet. Als Sprühtrocknungstemperatur wurde 130°C, als Sprührate 10%, als Aspiratorleistung 75% und als Flow control (Sprühdruck) 50% gewählt. Anschließend wurde das erhaltene Produkt mittels Röntgendiffraktogramm untersucht. Das Diffraktogramm ist in Figur 1 gezeigt.

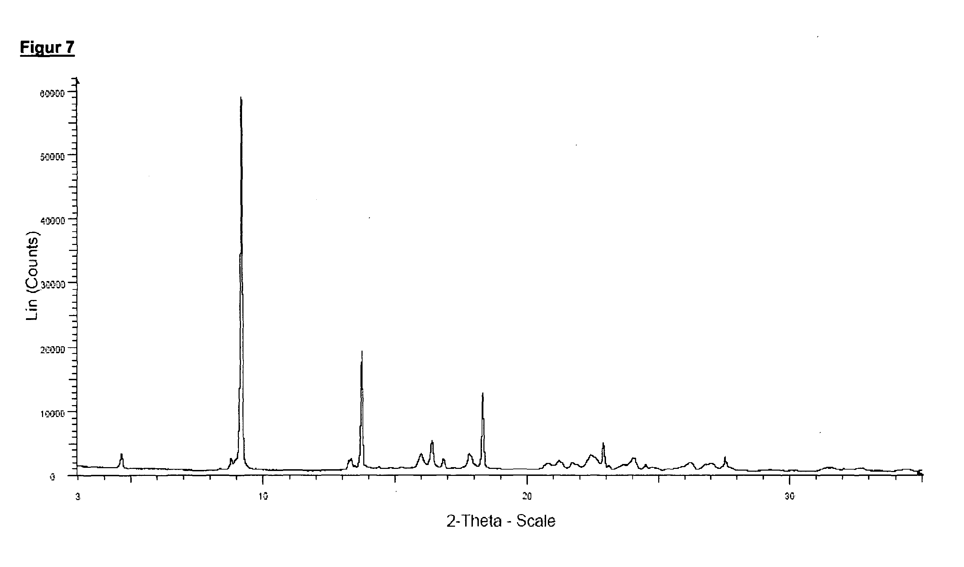

Es zeigen sich die üblichen Röntgenbeugungspeaks eines kristallinen Rasagilinmesylats. Als Vergleich ist ein typisches Rasagilinmesylat-Röntgendiffraktogramm in Figur 7 dargestellt.

### Beispiel 1

Adsorbate des Rasagilins mit verschiedenen Hilfsstoffen wurden gemäß folgender Tabelle hergestellt:

| **Beispiel** | **a** | **b** | **c** | **d** | **e** | **f** |
|---|---|---|---|---|---|---|
| Rasagilinmesylat | 1 g | 1 g | 1 g | 1 g | 1 g | 1 g |
| Kollidon 25 | 1 g | - | - | 9 g | - | - |
| HPMC | - | 1 g | - | - | 9 g | - |
| Zitronensäure | - | - | 1 g | - | - | 9 g |
| Avicel PH101 | - | - | 1 g | - | - | 9 g |
| Röntgendiffraktogramm | Figur 2 | Figur 3 | - | Figur 4 | Figur 5 | Figur 6 |

Bei den Versuchen gemäß Beispiel 1 a, 1 b, 1 d und 1 e wurde jeweils die in der vorstehenden Tabelle angegebene Menge an Rasagilinmesylat und die angegebene Menge an wasserlöslichem Hilfsstoff in 50 g Wasser in einem Becherglas gelöst und mit dem Sprühtrockner der Firma Büchi vom Typ B-191 sprühgetrocknet. Als Sprühtrocknungstemperatur wurde 130°C, als Sprührate 10%, als Aspiratorleistung 75% und als Flow control (Sprühdruck) 50% gewählt.

Bei den Versuchen 1c und 1f wurde zusätzlich die angegebene Menge Avicel PH 101 eingebracht. Die Versuchsdurchführung ist hier wie folgt: die in der vorstehenden Tabelle angegebene Menge an Rasagilinmesylat und die angegebene Menge an wasserlöslichem Hilfsstoff in 50 g Wasser in einem Becherglas gelöst. Anschließend erfolgte die Zugabe und Vermischung des wasserunlöslichen Hilfsstoffes Avicel PH 101, wie in der vorstehenden Tabelle beschrieben. Danach wurden die wässrigen Lösungen bzw. Gemische mit dem Sprühtrockner der Firma Büchi vom Typ B-191 sprühgetrocknet. Als Sprühtrocknungstemperatur wurde 130°C, als Sprührate 10%, als Aspiratorleistung 75% und als Flow control (Sprühdruck) 50% gewählt.

Kollidon 25 ist ein wasserlösliches Polyvinylpyrrolidon. Als Cellulosederivat wurde Hypromellose (HPMC) verwendet. Die verwendete Zitronensäure trägt als kommerziellen Produktnamen Zitronensäure, wasserfrei, Granulat.

Von den erhaltenen Adsorbaten wurden Röntgenbeugungsdiagramme aufgenommen, die in den Figuren gezeigt werden.

Es zeigt sich, dass in den Adsorbaten das Mesylatsalz des Rasagilins in amorphisierter Form vorliegt, die typischen Röntgenbeugungspeaks fehlen.

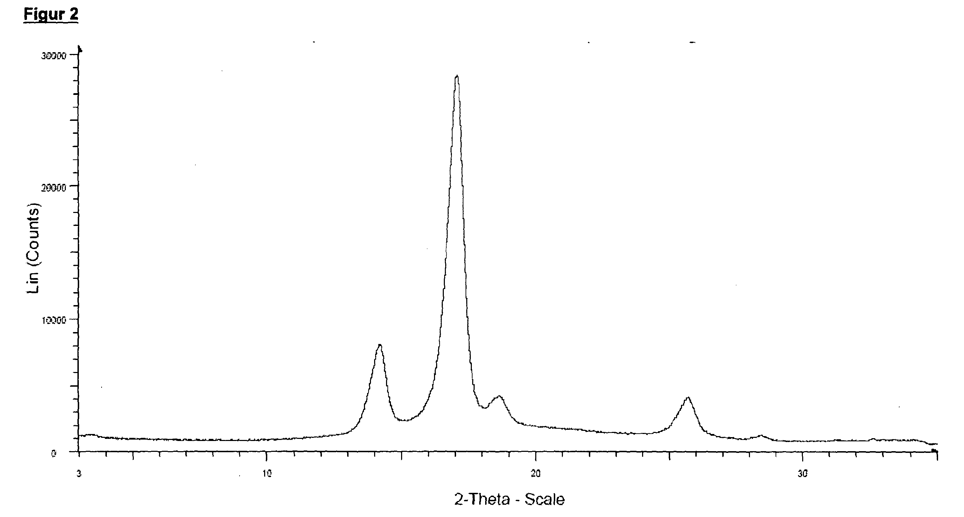

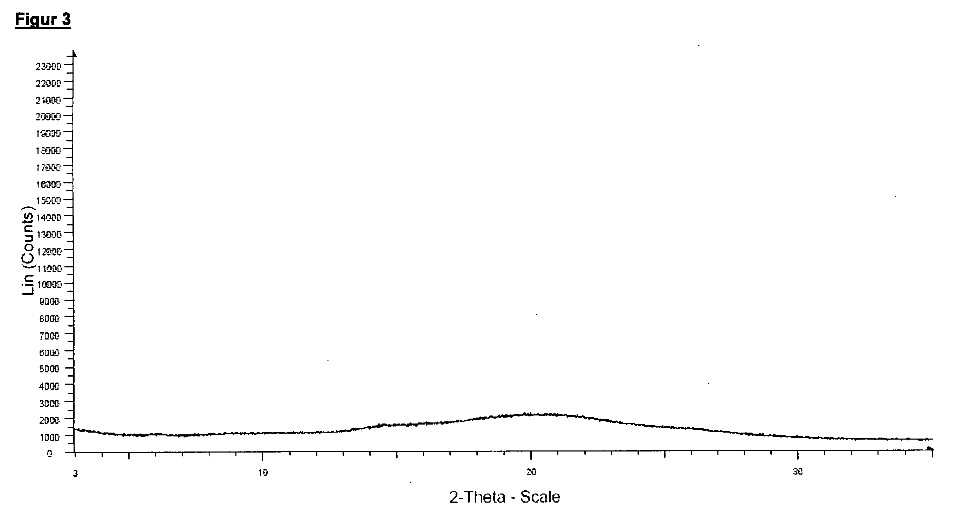

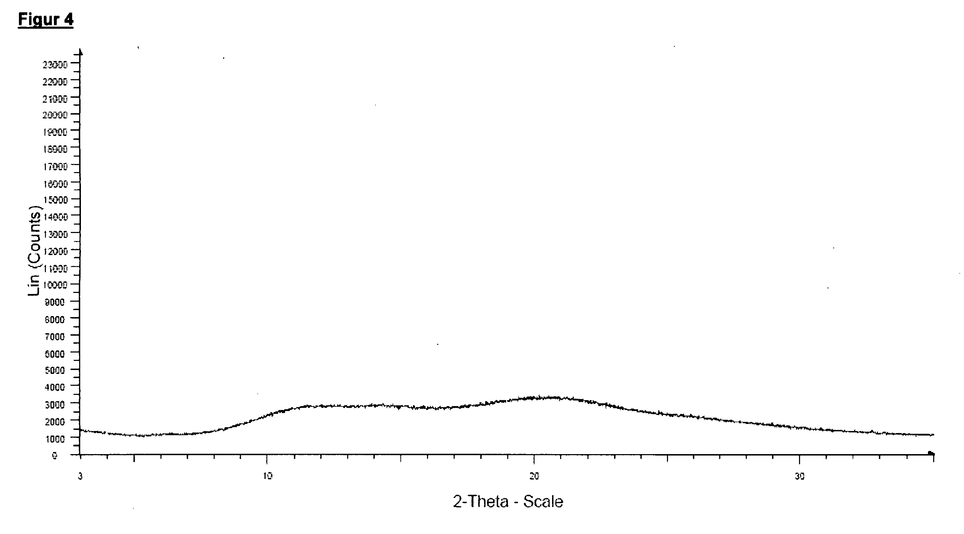

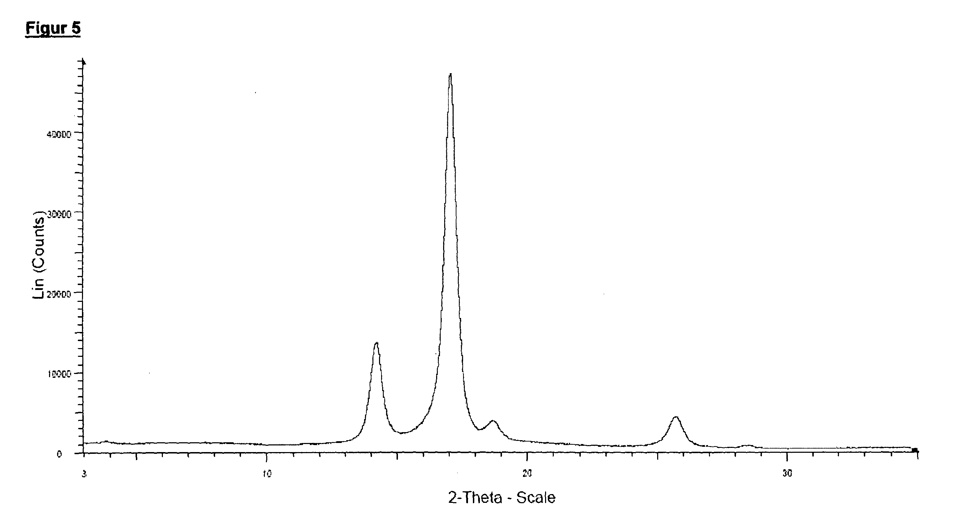

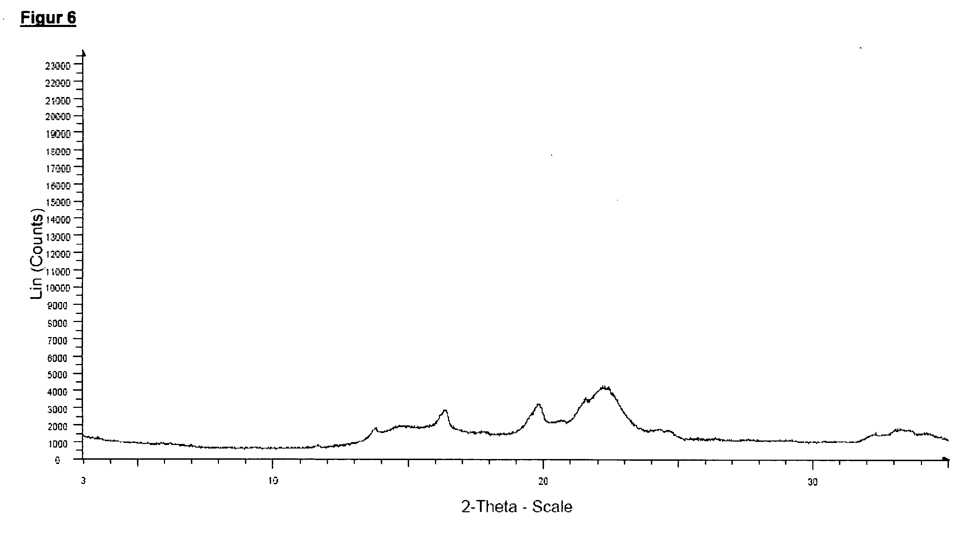

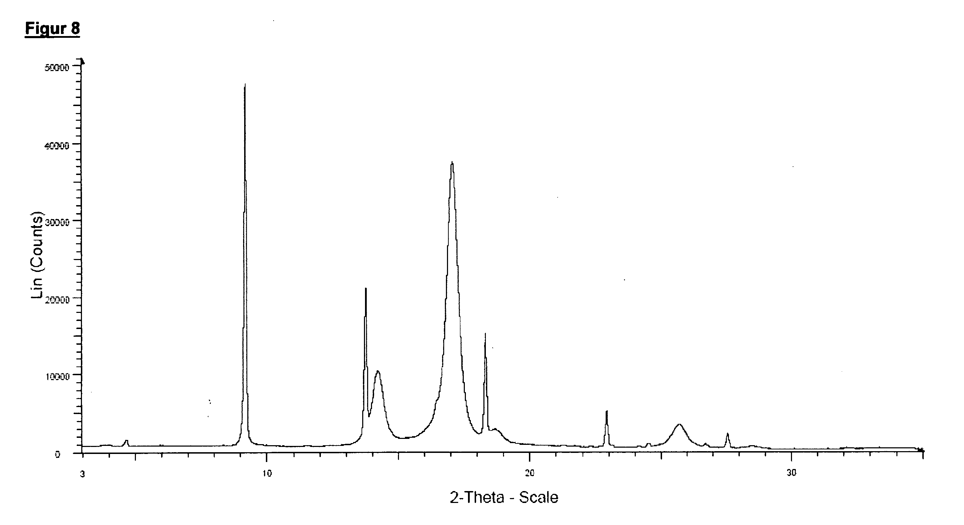

Beispiel 1a (Figur 2) wurde aufgrund geringer Menge an amorpher Substanz auf einen Träger aufgeklebt und untersucht. Die entstandenen Peaks sind auf den Träger zurückzuführen. Eine Untersuchung von kristallinem Rasagilinmesylat aufgebracht auf den gleichen Träger bestätigt dies (Figur 8).

### Beispiel 2

Beispiel 2 beschreibt ein ausgewähltes Herstellungsverfahren einer festen Arzneiform mit einem Rasagilinmesylat-Adsorbat des Beispiels 1d.

### Zusammensetzung:

| **Nr.** | **Substanz** | **Produkt** | **Funktion** | **[mg]** |
|---|---|---|---|---|
| 1 | Rasagilinmesylat-Adsorbat | | API | 3.27 |
| 2 | Avicel PH 101 | | Füllstoff | 184.80 |
| 3 | Starch 1500 | | Sprengmittel | 10.00 |
| 4 | Aerosil R972 | | Fließregulierungsmittel | 1.20 |
| 5 | Mg-Stearat | | Schmiermittel | 2.00 |
| 6 | **Total** | | | 201.27 |

### Herstellverfahren:

Rasagilinmesylat-Adsorbat, Avicel PH 101 und Starch 1500 einwiegen und im Turbula bei 23 U/ min für 5 min mischen. Anschließend die Mischung über ein Handsieb 0,5 mm sieben und 5 min mischen. Danach Aerosil R972 zugeben und 5 min mischen im Turbula. Die Mischung über ein Handsieb 0,5 mm sieben und 5 min mischen. Magnesiumstearat zugeben und 5 min im Turbula mischen. Mit EK0 zu 8 mm Kernen verpressen.

## Patentansprüche

1. Adsorbat eines pharmazeutisch verträglichen Salzes des Rasagilins mit zumindest einem pharmazeutisch verträglichen Hilfsstoff, wobei der zumindest eine pharmazeutisch verträgliche Hilfsstoff ein wasserlöslicher, organischer Hilfsstoff ist und das Salz des Rasagilins in dem Adsorbat als amorphe Substanz vorliegt.

2. Adsorbat nach Anspruch 1, wobei das pharmazeutisch verträgliche Salz des Rasagilins das Mesylatsalz ist.

3. Adsorbat nach Anspruch 1 oder 2, wobei das Adsorbat aus dem pharmazeutisch verträglichen Salz des Rasagilins und einem oder mehreren pharmazeutisch verträglichen, wasserlöslichen, organischen Hilfsstoffen besteht.

4. Adsorbat nach Anspruch 1 oder 2, wobei das Adsorbat aus dem pharmazeutisch verträglichen Salz des Rasagilins, einem oder mehreren pharmazeutisch verträglichen, wasserlöslichen, organischen Hilfsstoffen und einem oder mehreren pharmazeutisch verträglichen, wasserunlöslichen Hilfsstoffen besteht.

5. Adsorbat nach Anspruch 4, wobei ein pharmazeutisch verträglicher, wasserunlöslicher Hilfsstoff vorhanden ist und es sich bei diesem Hilfsstoff um mikrokristalline Cellulose handelt.

6. Adsorbat nach einem der vorstehenden Ansprüche, wobei der zumindest eine wasserlösliche, pharmazeutisch verträgliche Hilfsstoff ausgewählt ist aus wasserlöslichen, pharmazeutisch verträglichen Celluloseethern, wasserlöslichem Polyvinylpyrrolidon und wasserlöslichen, pharmazeutisch verträglichen, organischen Säuren.

7. Adsorbat nach Anspruch 6, wobei der zumindest eine wasserlösliche, pharmazeutisch verträgliche Hilfsstoff ausgewählt ist aus Hydroxypropylmethylcellulose, Hydroxypropylcellulose, Polyvinylpyrrolidon und Zitronensäure.

8. Adsorbat nach einem der vorstehenden Ansprüche, wobei das Verhältnis zwischen dem pharmazeutisch verträglichen Salz des Rasagilins und dem zumindest einen pharmazeutisch verträglichen, wasserlöslichen, organischen Hilfsstoff im Bereich von 5:1 bis 1:20 liegt.

9. Adsorbat nach Anspruch 8, wobei das Verhältnis zwischen dem pharmazeutisch verträglichen Salz des Rasagilins und dem zumindest einen pharmazeutisch verträglichen, wasserlöslichen, organischen Hilfsstoff im Bereich von 1:1 bis 1:10 liegt.

10. Verfahren zur Herstellung eines Adsorbats nach einem der Ansprüche 1 bis 9, bei dem das pharmazeutisch verträgliche Salz des Rasagilins und der zumindest eine pharmazeutisch verträgliche, wasserlösliche, organische Hilfsstoff in einem wässrigen Medium gelöst werden und anschließend das wässrige Medium entfernt wird.

11. Verfahren nach Anspruch 10, wobei das Adsorbat durch Sprühtrocknung einer Lösung des pharmazeutisch verträglichen Salzes des Rasagilins und des zumindest einen pharmazeutisch verträglichen, wasserlöslichen, organischen Hilfsstoffs hergestellt wird.

12. Arzneimittel, enthaltend ein Adsorbat nach einem der Ansprüche 1 bis 9 und gegebenenfalls ein oder mehrere pharmazeutisch verträgliche Hilfs- oder Zusatzstoffe.

13. Arzneimittel nach Anspruch 12, bei dem es sich um eine Kapsel, Tablette, im Mund zerfallende Tablette, verzögert freisetzende Tabletten, Pellets oder ein Granulat handelt.
